Europäisches Patentamt

European Patent Office

Office européen des brevets

(11) Publication number: **0 009 327**
**A1**

## (12) EUROPEAN PATENT APPLICATION

(21) Application number: **79301717.9**

(22) Date of filing: **22.08.79**

(51) Int. Cl.³: **A 61 B 17/18**

(30) Priority: **01.09.78 US 938861**

(43) Date of publication of application: **02.04.80**
**Bulletin 80/7**

(84) Designated Contracting States: **DE FR GB IT**

(71) Applicant: **Huddleston, Herbert D., 3704 Weslin Avenue, Sherman Oaks, California (US)**

(72) Inventor: **Huddleston, Herbert D., 3704 Weslin Avenue, Sherman Oaks, California (US)**

(74) Representative: **Kerr, Simonne June, c/o POTTS, KERR & CO. 27, Sheet Street, Windsor, Berkshire SL4 1BY (GB)**

(54) Hip nail and method for its use.

(57) The present invention relates to hip nails and methods for using them in the fixation of fractures about the upper end (25) of the femur. Prior art hip nails have been ineffective to consistently effect a rigid fixation sufficient to permit the patient to bear full body weight on the injured leg at an early stage of healing, without substantial pain, and without interfering with healing of the fracture. The present invention overcomes these difficulties by providing an improved hip nail and related method for its use for achieving a rigid fixation of a fracture of the upper femur. The hip nail includes an elongated nail portion (11), having a far end and a near end, along with a plate portion (17) coupled to the near end of the nail (11). The nail portion (11) is inserted into an elongated hole (13) extending from the laterial femoral cortex (15) across the fracture site to the interior of the femoral head (25), and an enlarged cavity (27) is formed at the far end of the hole for receiving a special cement material (29), which, when hardened, is pripped by the far end of the nail. The plate portion (17) is then attached to the femoral shaft by screws (45) and a flexible stainless steel band (41), to effect a rigid fixation of the fracture.

ACTORUM AG

## TECHNICAL FIELD

The present invention relates generally to orthopaedic implants for the fixation of fractured bones, and, more particularly, to hip nails and methods for using them in the fixation of fractures about the upper end of the femur.

## BACKGROUND OF THE PRIOR ART

The treatment of upper femoral fractures, including intertrochanteric and subtrochanteric fractures, has customarily been accomplished by a reduction of the bone fragments and surgical implanation of a hip nail to fix the fragments together. A number of techniques have been used in the past with a variety of nail designs. None of them, in the inventor's experience as a hip surgeon or to his knowledge, has been able to consistently effect a rigid fixation sufficient to permit the patient to bear full body weight on the injured leg at an early stage of healing, without substantial pain, and without interfering with healing of the fracture.

Most prior hip nail designs have included a metallic nail portion that extends from the lateral femoral cortex across the fracture site to the interior of the femoral head, along with an integral plate portion that is secured to the femoral shaft by screws. Fixation of the far end of the nail portion is ordinarily effected by friction between the nail and the femoral head fragment, aided by the tension of the muscles around the hip.

One popular technique has employed a Jewett nail, which has a sharpened end portion and a plurality of sharp, longitudinal fins. The nail is adapted to be inserted through a hole formed in the lateral femoral cortex and then forceably driven through the fractured bone fragments, into the femoral head. The fins engage both bone fragments, to hold the fragments fixed with respect to each other. After implantation of the Jewett nail, however, the sharpened end portion and the longitudinal fins can sometimes cut into the soft interior portion of the bone, enlarging the space occupied by the nail and thereby causing a loss of rigid fixation. Additionally, the nail can sometimes even cut through the surface of the bone into the hip joint. The nail is not generally strong enough to permit full weight bearing until healing of the bone has been substantially completed, so the patient ordinarily must avoid bearing weight on the injured leg for several months. Such prolonged periods of diminished physical activity are harmful for both psychological and physical reasons, the susceptibility of the patient to many diseases being heightened significantly.

Another popular technique has utilized a Richards nail, which is manufactured by Richards Surgical Manufacturing Company of Santa Monica, California, and which includes a plate for attachment to the femoral shaft, a cylindrical sleeve for insertion into an elongated hole projecting into the bone from the lateral femoral cortex, and a lag screw that is telescopically received within the sleeve. The threaded end of the screw projects outwardly from the far end of the sleeve to engage the interior of the femoral head. A separate machine screw is then used to engage a threaded bore in the head of the lag screw, and thereby to bring the bone fragments into compressive engagement with each other and couple the sleeve to the plate. Again, however, the sharp edges of the screw threads can sometimes cut into the soft interior portion of bone, enlarging the space occupied by the nail and causing a loss of rigid fixation, and can sometimes even cut through the surface of the bone into the hip joint. Additionally, the nail is not generally strong enough to permit full weight bearing.

Another technique of the prior art has utilized a Holt nail, which comprises an elongated cylinder having a blunt end. The nail is inserted into an elongated hole that has been drilled from the lateral femoral cortex across the fracture site to the interior of the femoral head. The Holt nail is substantially stronger than the Jewett or Richards nails, but it cannot by itself prevent relative rotational and telescopic movement of the bone fragments.

Another prior art technique is disclosed in U.S. Patent No. 3,255,747 to U. D. Cochran et al, and entitled "Apparatus for Treatment of Bone Fracture", wherein two fragments of a fractured bone are fixed to each other by an injection of polyurethane foam into a hole drilled across the fracture site. It is stated in the patent that the polyurethane foam completely fills the hole and expands into the lacunae of the bone structure, to form a rigid body for holding the fragments together. The polyurethane foam is the sole means for achieving fixation. In the disclosed technique, no attempt is made to prevent the polyurethane foam from entering the fracture site; rather, the foam is specifically forced into any interstices associated with the fracture. Additionally, it is stated that a short metal pin or tube can be embedded within the polyurethane foam, to provide supplemental strength for the main support member, i.e., the polyurethane foam. The disclosed pin or tube does not engage either of the bone fragments.

The Cochran et al technique has not proven satisfactory and is not in general use today, however, primarily because the polyurethane foam is absorbed into the bone too quickly and is not retained as a support structure for a sufficiently long duration to permit early weight-bearing by the patient, without bringing about a loss of rigid fixation or severe pain. Moreover, there are no known substitute materials for the polyurethane foam that, when used in this technique, will provide fixation without interfering with the healing of the fracture.

Still another prior technique for fixing fractures of the upper femur is described in an article entitled "The Use of Methylmethacrylate as an Adjunct in the Internal Fixation of Unstable Comminuted Intertrochanteric Fractures in Osteoporotic Patients", appearing in The Journal of Bone and Joint Surgery, September, 1975, pp. 744-750. The disclosed technique utilizes a special bone cement, methylmethacrylate, to supplement the fixation capability of hip nails of conventional design. Placement of the cement material is confined to the medullary canal (i.e., the marrow cavity), and it is specifically excluded from the femoral head. The cement does not cooperate directly with the hip nail to achieve improved fixation between the bone fragments, and the hip nails utilized in this technique function in the same manner as when used without the cement material. Accordingly, the technique is subject to many of the same drawbacks associated with those of more conventional techniques. Specifically, these drawbacks include excessive pain due to the sharp portions of the nails cutting into the soft interior portions of the bone, occasional loss of rigid fixation, and occasional cutting of the far end of the nail through the bone surface into the hip joint.

Another drawback associated with the use of prior hip nails arises from a tendency of the screws holding the plate portion of the nail to the femoral shaft to break, whenever the patient is allowed full weight bearing. Such breakage can cause loss of fixation of the fracture fragments.

When an animal suffers a severe bone fracture of a type for which humans would customarily be treated by the surgical implantation of a nail or rod, it is usually killed, since a strict limitation on the animal's use of the injured bone cannot ordinarily be practically imposed. This is especially the case when the bone is in a weight-bearing limb. A loss of rigid fixation of the fracture, along with severe pain almost inevitably result from the practical inability to restrict the animal's physical activity. Hence, there is a need for a technique that will permit a prompt use by the animal of its injured bone, without causing a loss of fixation and without causing significant pain.

It will be appreciated from the foregoing that there is a definite need for a hip nail, along with a method for its use, that can consistently effect a rigid fixation of fractured bone fragments and that can permit early weight bearing by the patient, without significant pain. The present invention fulfills this need.

## BRIEF SUMMARY OF THE INVENTION

This invention resides in an improved hip nail, and a special method for its use, for rigidly fixing fractures of the upper femur, including intertrochanteric and subtrochanteric fractures. A nail is inserted into an elongated hole that is formed in the fractured bone and that extends across the fracture site, from a near bone fragment to far bone fragment. In accordance with the invention, a portion of the elongated hole in the far bone fragment is adapted to receive a special cement material, such as methylmethacrylate, and the nail includes means for gripping the cement, to thereby rigidly secure the nail to the fragment. The nail can then be secured to the near bone fragment by any suitable technique, such as screwing an integral plate into the fragment, whereby a rigid fixation of the fracture is achieved. As a result, early weight bearing by the patient, without significant pain, is facilitated and long periods of severely limited physical activity can be avoided.

More specifically, the near fragment normally includes the femoral shaft and the far fragment normally includes the femoral head. The elongated hole extends from the lateral femoral cortex across the fracture site to the interior of the femoral head, where the cement is deposited. A hip nail constructed in accordance with the present invention has an elongated nail portion for placement in the elongated hole, and an integral plate portion for attachment to the femoral shaft. The means for gripping the cement, which is carried on the far end of the nail portion, can be formed in the nail portion itself and can include, for example, asymmetry in both its longidutinal and circumferential axes, such as a longitudinal channel and a circumferential groove in the exterior surface of the nail, or, alternatively, surface texture or roughness. The plate portion is then attached to the femoral shaft, and relative rotational and

-6-    0009327

telescopic movement of the nail and the bone fragments is precluded. The nail portion is preferably formed of a strong alloy material such as Vitalium and is preferably free of any sharp edges, which might cut into the hardened cement.

In accordance with one aspect of the invention, the cement is received in an enlarged cavity formed at the far end of the elongated hole, in the interior of the femoral head, and the hip nail can include means for channeling the flow of air and excess cement out of the cavity when the nail is inserted. This channeling means can take the form, for example, of a longitudinal channel extending over the length of the exterior surface of the nail, or, alternatively, a longitudinal hole extending over the length of the nail. Care must be taken to insure that the excess cement is not permitted to flow into the fracture site, an occurrence that could inhibit union of the bone fragments.

The enlarged cavity preferably has an asymmetric shape, so that the hardened cement is held rigidly within the cavity. Additionally, the cavity is preferably larger in its horizontal dimension than its vertical dimension, so that stresses on the soft interior portion of the bone during weight bearing are minimized.

In an alternative embodiment of the present invention, the hip nail can include a special end element, which is removably attached to the far end of the nail portion. When the nail is in use, the end element is disposed within the enlarged cavity and is thus gripped by the cement. If the nail must later be removed from the bone, as, for example, would occur if the patient later developed severe arthritis and a total hip replacement were necessitated, the nail portion can be detached from the end element and the nail removed from the hole. In particular, the end element can comprise a nut, which is engageable with a screw disposed in a longitudinal hole that extends the length of the nail. The nut, which is held against rotation by the cement, can then be detached from the nail by simply rotating the screw.

In accordance with still another aspect of the present invention, the plate portion of the hip nail can be attached to the femoral shaft by means of a flexible band, such as a Parham band, in addition to conventional screws. The band, which is made to encircle both the femoral shaft and the plate, can be disposed in a groove or seat formed in the plate, thereby preventing the band from sliding axially along the shaft.

Many other aspects and advantages of the present invention will become apparent from the following description of the preferred embodiments, taken in conjunction with the accompanying drawings, which disclose, by way of example, the principles of the invention.

## BRIEF DESCRIPTION OF THE DRAWINGS

In the accompanying drawings:

FIG. 1 is a sectional view of a hip nail embodying the present invention, the nail being shown in operative position holding the fragments of a fractured hip bone in rigid fixation;

FIG. 2 is an elevational view of the hip nail of FIG. 1, taken substantially in a direction normal to the plate portion thereof;

FIG. 3 is a sectional view of the hip nail, taken substantially along the line 3-3 in FIG. 1;

FIG. 4 is a plan view of a Parham band, which can be utilized to secure the plate portion of the hip nail of FIG. 1 to the femoral shaft;

FIG. 5 is a perspective view of an alternative embodiment of a hip nail in accordance with the present invention, the far end of the nail portion thereof having a textured surface for gripping the cement material;

.FIG. 6 is an exploded partial perspective view of still another embodiment of a hip nail in accordance with the present invention, wherein the nail includes an irregularly-shaped end element removably attached to the far end of the nail portion thereof; and

FIG. 7 is a sectional view of a portion of the hip nail of FIG. 6, showing the removable end element disposed in its operative position in a cement-filled cavity formed in the femoral head.

## DETAILED DESCRIPTION OF THE INVENTION

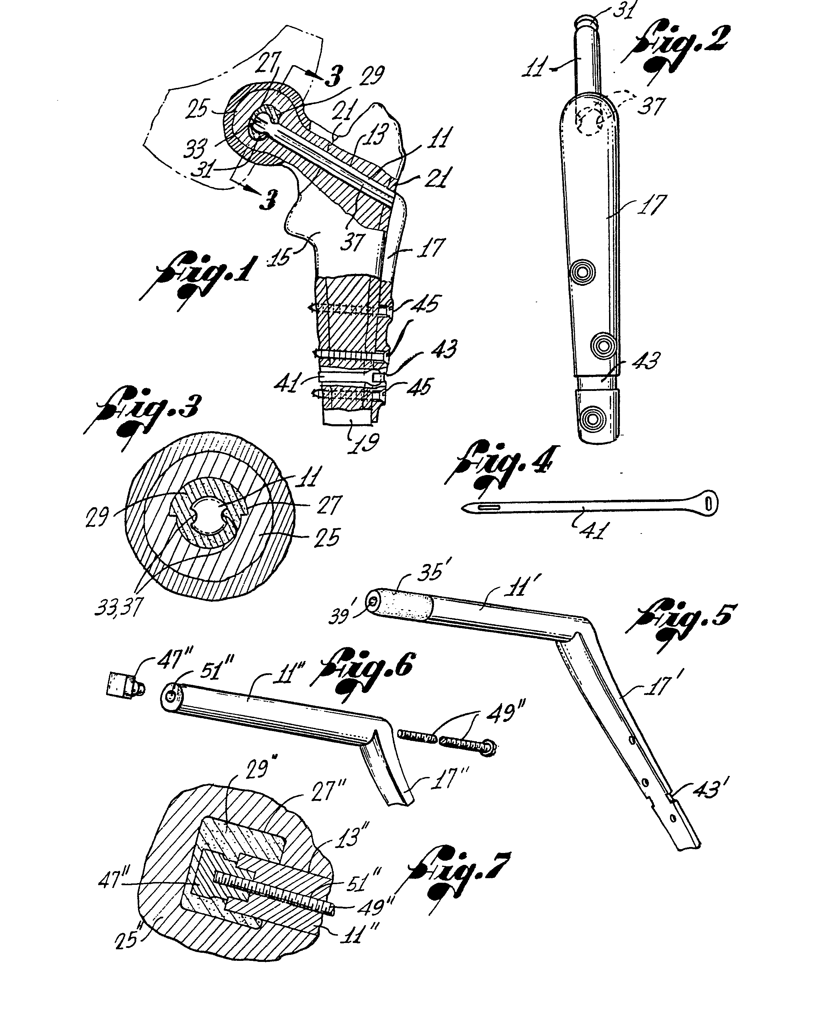

Referring now to the drawings, and particularly to FIGS. 1 through 3, there is shown a hip nail constructed in accordance with one preferred embodiment of the present invention. The hip nail includes a generally cylindrical nail portion 11, which in use is disposed in an elongated hole 13 formed in a fractured hip bone 15, along with an integral plate portion 17, which in use is secured to the femoral shaft 19. The hole 13 extends from the lateral femoral cortex 21 across the fracture site 23 to the interior of the femoral head 25.

In accordance with the invention, an enlarged cavity 27 is formed at the far end of the hole 13 for receiving a special cement material 29, which can be inserted by means of a syringe (not shown). The nail portion 11 of the hip nail is inserted into the hole before the cement has hardened, and the far end of the nail portion includes means for gripping the cement. By this technique, after the cement has hardened, relative movement of the nail and the femoral head 25 will be prevented. After the cement has hardened, the plate portion 17 of the hip nail is secured to the femoral shaft 19 and a rigid fixation of the hip bone fragments is achieved, thereby promoting a rapid union of the bone fragments.

In the embodiment shown in FIGS. 1 through 3, the means for gripping the cement 29 take the form of an annular groove 31 encircling the nail portion 11 of the hip nail, near its far end, and a pair of longitudinal grooves 33 located between the annular groove 31 and the far end of the nail. When the nail is inserted into the hole 13, the cement fills the grooves, whereupon after the cement has hardened, relative rotational and telescopic movement of the nail is prevented.

One cement material 29 that has been used advantageously is methylmethacrylate, which is initially inserted into the cavity 27 in a soft, putty-like form, but which hardens within about ten minutes to form a rigid structure. Methylmethacrylate has no real adhesive

properties, but rather, functions as a hard filler material that conformably envelopes the gripping means on the end of the hip nail to prevent relative movement thereof.

The hip nail is preferably formed of a strong alloy material such as Vitalium and is preferably free of any weakening structural irregularities. This facilitates early weight bearing by the patient, and, since the cement 29 operates to hold the bone fragments in rigid fixation, prolonged periods of severely-restricted physical activity can be obviated. Additionally, the nail portion 11 of the hip nail is preferably free of any sharp edges, which might cut into either the hardened cement 29 or the soft interior portion of the bone surrounding the hole 13. As a result, pain is substantially reduced and the likelihood of rigid fixation is substantially improved.

The enlarged cavity 27 formed at the far end of the hole 13 preferably has an asymmetric shape, thereby insuring that relative movement of the cement 29 and the femoral head 25 is prevented. Additionally, the cavity is preferably larger in its horizontal dimension (when the patient is erect) than its vertical dimension, so that forces applied to the soft interior portion of the bone during weight-bearing are better distributed.

In accordance with one aspect of the invention, the hip nail also includes means for channeling the flow of air and excess cement 29 out of the cavity 27 when the nail portion 11 is inserted therein. In the embodiment of FIGS. 1 through 3, this channeling means takes the form of a pair of longitudinal channels 37 formed along the length of the exterior of the nail portion. Since the presence of cement material in the region of the fracture site 23 can inhibit union of the bone fragments, it is preferred that the amount of excess cement be limited so that it does not flow back along the channels 37 as far as the fracture site. Additionally, it has been found that the hole 13 can be sized to be slightly greater in diameter than the nail portion 11 of the hip nail, whereby a portion of the excess cement 29 can flow into the region

between the nail and the hole.

After the hip nail has been placed in its operative position and the cement 29 has hardened, the plate portion 17 is secured to the femoral shaft 19. In accordance with another aspect of the invention, the plate can be secured by means of a strong flexible band such as a stainless steel Parham band 41, which encircles both the plate 17 and the shaft 19, along with conventional screws 45. Use of the band avoids problems that can sometimes arise when screws alone are used and the heads of the screws snap off from corrosion and high stress.

The Parham band 41 is preferably disposed in a special groove 43 formed at the lower end of the plate 17, thereby preventing the band from sliding axially along the femoral shaft 19. After attachment of the plate portion of the hip nail to the femoral shaft, a rigid fixation of the bone fragments is achieved.

In the drawings, FIGS. 1 through 3 show a first embodiment of the present invention, FIG. 5 shows a second embodiment, and FIGS. 6 and 7 show a third embodiment. In each embodiment, corresponding reference numerals are utilized to identify corresponding elements, with the numerals for the embodiment of FIGS. 1 through 3 being unprimed, the numerals for FIG. 5 being singly primed (i.e., '), and the numerals for FIGS. 6 and 7 being doubly primed (i.e., ").

In the second embodiment of the present invention, shown in FIG. 5, the means for gripping the cement comprises a surface texture 35' adjacent the far end of the nail portion 11' of the hip nail. The cement material conformably envelopes the textured surface, whereby after it is hardened it is gripped by the nail and relative rotational and telescopic movement are prevented. Although the hip nail of FIG. 5 grips the cement 29' with less holding force than does the hip nail of FIGS. 1 through 3, it has been found that the magnitude of this gripping force is sufficient to preclude relative movement during ordinary circumstances.

In the embodiment of FIG. 5, the means for channeling the flow of air and excess cement 29' is shown in the form of a longitudinal bore hole 39' extending the length of the nail portion 11'. The cement material may be too viscous to flow readily through the bore hole 39', so the hole 13 in the bone 15 can be appropriately sized to permit at least a portion of the excess cement to flow into the region between the hole and the nail 11'. Again, however, care must be taken to insure that the cement does not flow into the fracture site 23. Alternatively, the bore hole 39' can be made large enough to allow excess cement to readily escape.

FIGS. 6 and 7 depict still another embodiment of the present invention. In this embodiment, the means for gripping the cement comprises a removable element in the form of a nut 47" that is removably attached to the end of the nail portion 11" of the hip nail. When the hip nail is inserted into the hole 13" formed in the hip bone 15", the nut is disposed in the cavity 27", where it grips the cement 29" and prevents relative rotational and telescopic movement of the nail. The nut is held in place at the end of the nail by means of a screw 49", which is disposed in a longitudinal bore hole 51" and extends the length of the nail.

If, after its surgical implantation, the hip nail must be removed from the hip bone, as would occur if the patient develops severe arthritis and the femoral head 25" must be excised and replaced, the screw 49" can be rotated to disengage the nut 47" from the nail and the nail can then be removed from the bone 15". With the nail removed, excision and replacement of the femoral head can then be accomplished using conventional surgical techniques.

From the foregoing description, it should be apparent that the present invention provides an improved hip nail and a method for utilizing it in combination with a special cement material to achieve a rigid fixation of an upper femoral fracture. The invention permits early and relatively pain-free use of the injured bone by the patient, so prolonged periods of severely limited physical activity are obviated.

While particular forms of the invention have been illustrated and described, it will be understood by one of ordinary skill in the art that various modifications can be made without departing from the spirit and scope of the invention. Accordingly, it is not intended that the invention be limited other than by the appended claims.

CLAIMS

1. A method for the surgical fixation of a bone fracture, comprising the steps of: forming an elongated hole in the bone, said hole extending from a near bone fragment across the fracture site into a far bone fragment; enlarging a portion of the hole in the far bone fragment to form a cavity; depositing a cement material in said cavity; inserting an elongated nail into said hole before the cement material has hardened, whereby after the cement material has hardened a first portion of said nail cooperates with the hardened cement to rigidly fix the nail to the far bone fragment; and securing a second portion of said nail to the near bone fragment, whereby a rigid fixation of the bone fragments is achieved.

2. The method as claimed in Claim 1, wherein: said step of forming an elongated hole includes the step of drilling into the bone; and said step of enlarging includes the step of curetting a portion of the soft interior of the far bone fragment.

3. The method as claimed in Claim 1, wherein: said step of inserting an elongated hip nail includes the step of channeling air and excess cement material out of said cavity.

4. The method as claimed in Claim 1, wherein: when the fracture is about the upper femur, the elongated hole extends completely through the near bone fragment; and the cavity formed in said step of enlarging is disposed at the far end of the elongated hole, in the interior of the femoral head.

5. The method as claimed in Claim 4, wherein: the cavity formed in said step of curetting has a horizontal dimension greater than its vertical dimension, thereby reducing stresses imparted on the soft interior portion of the far bone fragment when the patient is erect.

6. The method as claimed in Claim 4, wherein: said step of securing includes the step of attaching a plate to the femoral shaft.

7. A hip nail for use in combination with a cement material for the surgical fixation of a bone fracture, comprising: an elongated nail having a far end and a near end for placement in an elongated hole extending from a near bone fragment across the fracture site to the interior of a far bone fragment, a portion of said elongated hole in the far bone
fragment being adapted to receive the cement material; gripping
means associated with the far end of said elongated nail for
gripping the cement material; and means for securing the near
end of said elongated nail to the near bone fragment, whereby
after the cement material has hardened, a rigid fixation of
the respective bone fragments is achieved.

8.    The hip nail as claimed in Claim 7, wherein:
said elongated nail and said gripping means are substantially
free of sharp edges that can cut into the hardened cement material
or the soft interior portions of the bone fragments.

9.    The hip nail as claimed in Claim 7, wherein:
said gripping means includes a longitudinal groove formed in
the exterior surface of said elongated nail.

10.    The hip nail as claimed in Claim 9, wherein:
said gripping means further includes an annular groove formed
in the exterior surface of said elongated nail.

11.    The hip nail as claimed in Claim 7, wherein:
said gripping means includes a rough texture on the exterior
surface of said elongated nail.

12.    The hip nail as claimed in Claim 7, further
including:  means for channeling air and excess cement material
from the portion of the elongated hole adapted to receive the
cement when said elongated nail is inserted therein.

13.    The hip nail as claimed in Claim 12, wherein:
said channeling means includes a longitudinal channel formed
in the exterior surface of said elongated nail, said channel
extending from a location near the far end of the nail to the
near end of the nail.

14.    The hip nail as claimed in Claim 7 for the
surgical fixation of a fracture of the upper femur, wherein:
said elongated hole extends from the lateral femoral cortex
across the fracture site to the interior of the femoral head,
an enlarged cavity being formed at the far end of said hole to
define said portion of said elongated hole adapted to receive
the cement material; said gripping means associated with the
far end of said elongated nail for gripping the cement material
thereby fixes the nail to the femoral head; and said means for
securing the near end of said elongated nail secures said near end to the femoral shaft.

15. The hip nail as claimed in Claim 14, wherein: said gripping means includes an element removably attached to the far end of said elongated nail.

16. The hip nail as claimed in Claim 14, wherein: the elongated hole extends completely through the near bone fragment; and said securing means includes a plate coupled to the near end of said elongated nail, and means for securing said plate to the femoral shaft.

17. The hip nail as claimed in Claim 15, wherein: said removable element is a nut; said elongated nail is formed with a longitudinal through-bore extending the length thereof; and said hip nail further includes a screw, a portion of said screw disposed within said through-bore and engageable with said nut, whereby after said cement material has hardened, said screw can be disengaged from said nut and the elongated nail removed from said elongated hole.

18. The hip nail as claimed in Claim 14, wherein: said enlarged cavity has a greater horizontal dimension than a vertical dimension, whereby stresses on the soft interior portion of the femoral head encountered during weight bearing are reduced.

Fig.1

Fig.2

Fig.3

Fig.4

Fig.5

Fig.6

Fig.7

## PARTIAL EUROPEAN SEARCH REPORT

European Patent Office

which under Rule 45 of the European Patent Convention shall be considered, for the purposes of subsequent proceedings, as the European search report

### DOCUMENTS CONSIDERED TO BE RELEVANT

| Category | Citation of document with indication, where appropriate, of relevant passages | Relevant to claim |
|---|---|---|
| | GB - A - 1 429 633 (NATIONAL RE-SEARCH DEVELOPMENT CORPORATION) <br> * Page 2, lines 39-68; figures * | 7,8,12 14,16 |
| D | US - A - 3 255 747 (COCHRAM) <br> * Figures 2,2a; column 4, line 64 - column 5, line 19 * | 7,14 |
| | GB - A - 1 315 220 (FISCHER) <br> * Figures; claim 1 * | 7,8,14 |
| A | US - A - 4 095 591 (GRAHAM) <br> * Figures; column 1, line 66 - column 2, line 26 * | 7,14 |
| A | FR - A - 590 290 (LAUWENS) <br> * Figure 1; page 1, line 58 - page 2, line 10 * | 16 |

**CLASSIFICATION OF THE APPLICATION (Int. Cl.³)**

A 61 B 17/18

**TECHNICAL FIELDS SEARCHED (Int. Cl.³)**

A 61 B

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely:

Claims not searched:

Reason for the limitation of the search:

1-6: in view of article 52(4) of the European Patent Convention.

**CATEGORY OF CITED DOCUMENTS**

X: particularly relevant

A: technological background

O: non-written disclosure

P: intermediate document

T: theory or principle underlying the invention

E: conflicting application

D: document cited in the application

L: citation for other reasons

&: member of the same patent family, corresponding document

| Place of search | Date of completion of the search | Examiner |
|---|---|---|
| The Hague | 06-12-1979 | STEENBAKKER |

EPO Form 1505.1 06.78